# EUROPEAN PATENT APPLICATION

(11) **EP 2 514 461 A1**
(43) Date of publication of application: **24.10.2012**
(21) Application number: 11163250.1
(22) Date of filing: 20.04.2011
(51) Int. Cl.: A61M 5/34

(54) **Needle assembly for a medical device**

(71) Applicant: Sanofi-Aventis Deutschland GmbH, 65929 Frankfurt (DE)
(72) Inventor: The designation of the inventor has not yet been filed

(57) **Abstract**

Described is a needle assembly (A) comprising a needle hub (1) having a coupling mechanism adapted to engage corresponding coupling mechanism of a retainer (2), and a notch (6) formed in a proximal end of the needle hub (1), wherein the notch (6) is adapted to engage a projection (5) on the retainer (2).

## Description

### Technical Field

The invention relates to a needle assembly for a medical device. More particularly, the invention relates to a needle assembly for an injection device.

### Background of the Invention

Conventional needle assemblies adapted to connect a retainer for a medical ampoule are well known. Typically, the needle assembly comprises a screw thread that engages a corresponding screw thread formed on the retainer. However, patients may over-tighten the needle assembly to the retainer which can lead to removal problems. Similarly, over-tightening (or attempts to replace an over-tightened needle assembly) may lead to displacement of portions of the retainer or mechanisms in the retainer used for administering a dose of medicament or needle-stick injuries.

Thus, there is a need for a needle assembly which prevents the problems associated with over-tightening the needle assembly to a medical device.

### Summary of the Invention

It is an object of the present invention to provide an improved needle assembly suitable for a retainer (e.g., an injection device) containing a medical ampoule that allows for a quick, easy and safe fastening and unfastening. It is a further object of the invention to provide a method of assembling a medical device that avoids jamming of the assembled parts.

In an exemplary embodiment, a needle assembly comprises a needle hub having a coupling mechanism adapted to engage corresponding coupling mechanism of a retainer, and a notch formed in a proximal end of the needle hub. The notch is adapted to engage a projection on the retainer. A shape of the projection corresponds to a shape of the notch.

In an exemplary embodiment, a feedback is provided when the projection engages the notch. The feedback may be at least one of a tactile feedback and an audible feedback.

In an exemplary embodiment, a plurality of notches may be formed in the proximal end of the needle hub, and a plurality of projections may be formed on the retainer. The plurality of projections may be disposed at regular intervals.

The notch may be an inverted U-shape or triangular.

In an exemplary embodiment, the notch includes a surface adapted to prevent movement of the projection in a first direction.

The exemplary embodiments of the needle assembly according to the present invention are preferably used in combination with retainers that may be used as components of injection devices, including but not limited to, pen-type injection devices, syringes, cartridge syringes, safety syringes, auto-injectors, etc. Further, it is foreseen that the needle assembly according to the present invention may be utilized with catheter and/or infusion assemblies.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows a sectional view of a prior art needle assembly;
- Figure 2: shows an isometric view of a needle assembly according to an exemplary embodiment of the invention;
- Figure 3: shows an isometric view of a needle assembly according to an exemplary embodiment of the invention;
- Figure 4A and 4B: show isometric views of a needle assembly according to an exemplary embodiment of the invention;

Corresponding parts are marked with the same reference symbols in all figures.

### Detailed Description

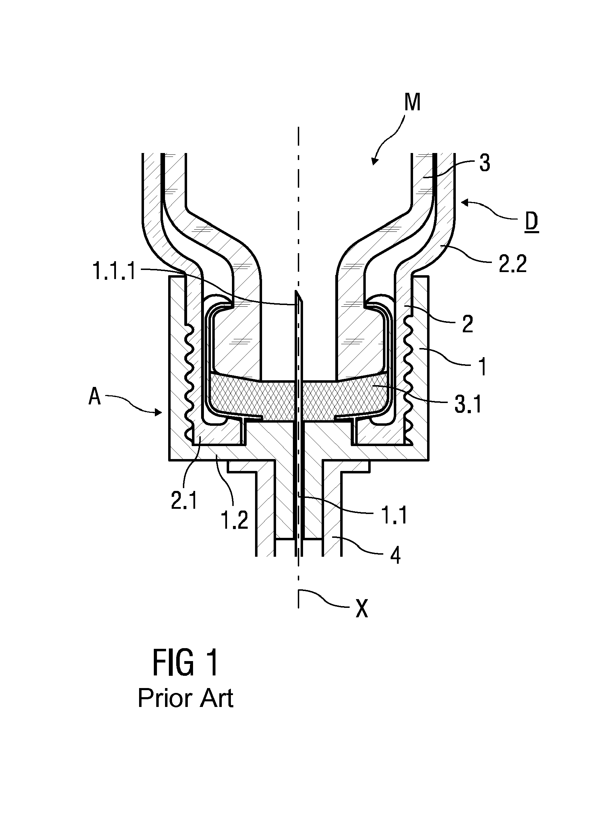

Figure 1 shows a conventional needle assembly A for a medical device D according to the prior art. The conventional needle assembly A consists of a needle hub 1 with a double-sided needle 1.1 and a retainer 2 for housing a medical ampoule 3 containing a medicament M. A distal opening of the medical ampoule 3 is sealed by a septum 3.1. The needle 1.1 has a distal tip for penetrating the skin of a patient and a pointed proximal tip 1.1.1 for piercing the septum 3.1 when the needle hub 1 is mounted to the retainer 2 so as to establish fluid communication of the needle 1.1 with the interior of the medical ampoule 3.

Corresponding screw threads are formed on an inner surface of the needle hub 1 and an outer surface of a distal section of the retainer 2 so that the needle hub 1 may be engaged with the retainer 2. When the needle hub 1 is properly engaged with the retainer 2, further rotation of the needle hub 1 relative to the retainer 2 is blocked by (i) a distal surface 2.1 of the retainer 2 abutting against a distal surface 1.2 of the needle hub 1 and (ii) a proximal end of the needle hub 1 abutting against a shoulder 2.2 of the retainer 2. However, the user may continue attempting to rotate the needle hub 1 relative to the retainer 2 which can result in difficulty removing the needle hub 1, injury when removing the needle hub 1, or displacement of other components of the retainer 2 (or mechanisms therein for dispensing the medicament M).

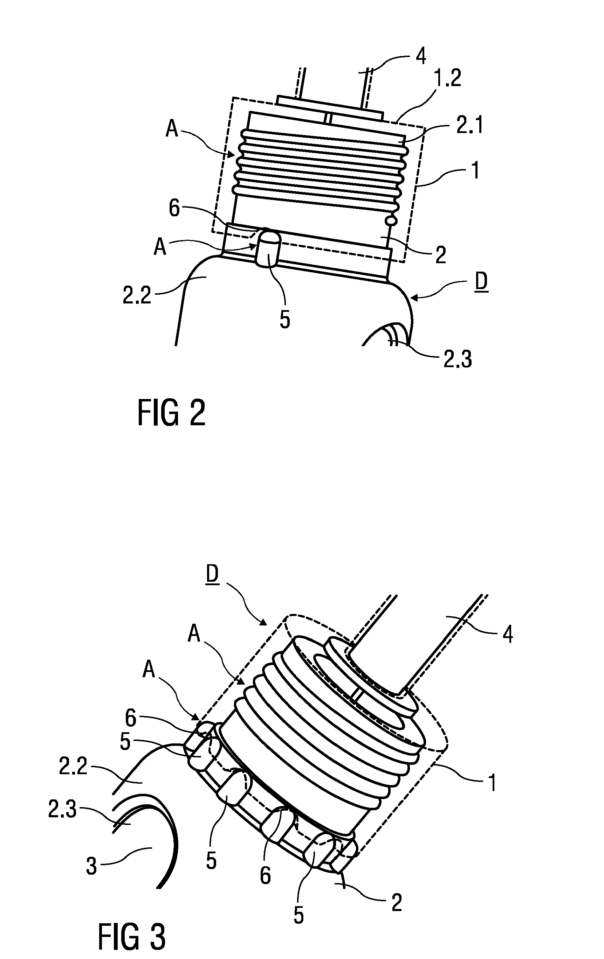

Figure 2 shows an isometric view of an exemplary embodiment of a needle assembly A according to the present invention. The needle assembly A comprises a needle hub 1 with a coupling mechanism (e.g., screw threads, a bayonet fitting, a frictional surface, etc.) formed on an inner surface thereof. The coupling mechanism on the needle hub 1 is adapted to engage a corresponding coupling mechanism (e.g., screw threads, a bayonet fitting, a frictional surface, etc.) formed on an outer surface of a distal end of a retainer 2 for a medical ampoule 3. In an exemplary embodiment, the needle hub 1 may be cylindrical and include a textured surface on an outer surface thereof to facilitate the user's grip when coupling to and removing from the retainer 2. However, those of skill in the art will understand that the needle hub 1 may be of any shape and size which will suitably accommodate a distal end of the retainer 2. The retainer 2 may be a part of a pen-type injection device, a syringe, a cartridge syringe, a safety syringe, an auto-injector, a catheter and/or any other injection device and/or infusion assembly.
The needle assembly A further includes a needle 1.1 coupled to the needle hub 1.
In the exemplary embodiment, the needle 1.1 includes a distal tip for piercing the skin and a proximal tip for piercing a septum of the medical ampoule 3.

In an exemplary embodiment, the needle hub 1 includes at least one notch 6 formed in its proximal end. For example, as shown in the exemplary embodiment in Figure 2, the notch 6 is formed as an inverse U-shaped recess in a circumference of the proximal end of the needle hub 1. While the exemplary embodiment shown in Figure 2 depicts the needle hub 1 having one notch 6, those of skill in the art will understand that the needle hub 1 may include a plurality of notches 6, as shown in the exemplary embodiment in Figure 3. The plurality of notches 6 may be spaced at regular intervals around a circumference of the distal end of the retainer 2.

The notch 6 may be sized and shaped to engage a projection 5. In an exemplary embodiment, the projection 5 may be formed on an outer surface of a distal end of the retainer 2, proximal to the screw threads formed on the retainer 2. For example, the projection 5 may be formed as a protrusion on a shoulder 2.2 of the retainer 2 and extend a predetermined distance in the distal direction. In an embodiment in which there is a plurality of projections 5, the projections 5 may extend the same or different distances in the distal direction. The projection 5 may include a proximal end which abuts the shoulder 2.2 and a distal end which is sized and shaped to engage the notch 6. For example, in the exemplary embodiment in which the notch 6 is an inverted U-shaped recess, the projection 5 may have an inverted U-shaped distal end. The projection 5 may extend a predetermined distance distally along a length of the outer surface of the distal end of the retainer 2. Preferably, the shapes of the projection 5 and 6 correspond closely such that use of the medical device D during an injection does not cause a projection 5 to rotate past a corresponding notch 6.

In another exemplary embodiment, the notch 6 may be formed on the shoulder 2.2 of the retainer 2, and the projection 5 may be formed on the proximal end of the needle hub 1. In another exemplary embodiment, the projection 5 may be formed on a coupling which attaches to the retainer 2. That is, the coupling may be attached to a distal end of an otherwise conventional medical device D to allow the medical device D to accommodate a needle hub 1 according to the present invention.

When the needle assembly A is attached to the retainer 2, screw threads on the needle hub 1 may engage corresponding screw threads on the retainer 2. Prior to the screw threads and the corresponding screw threads being substantially fully engaged, the user may receive a first feedback (e.g., a resistance) which indicates that a distal portion of the projection 5 is abutting a proximal portion of the needle hub 1. As the needle hub 1 is further rotated, a second feedback (e.g., sudden release of resistance and/or an audible sound) may be provided to the user when the projection 5 engages the notch 6. The second feedback may indicate to the user that the needle hub 1 has properly engaged the retainer 2 and that further rotation is neither necessary nor desirable.

In another exemplary embodiment, the notch 6 may be formed such that it prevents rotation of the needle hub 1 in a tightening direction after the needle hub 1 has been properly engaged by the retainer 2. For example, the notch 6 may be a triangular recess which has a first leg sloped relative to the proximal end of the needle hub 1 and a second leg perpendicular relative to the proximal end of the needle hub 1. In this exemplary embodiment, when the projection 5 engages the notch 6, the projection 5 may abut the second leg, preventing further rotation of the needle hub 1 relative to the retainer 1 in a first, tightening direction. However, the first leg would not prevent rotation in a second, loosening direction, allowing for removal of the needle hub 1.

When the projection 5 protrudes into the notch 6, the needle hub 1 may be properly secured to the retainer 2. In an exemplary embodiment, the projection 5 and the notch 6 are dimensioned so as to define an axial gap between the first and second distal surfaces 2.1, 1.2 when the needle hub 1 is screwed all the way in and the relative rotation of the needle hub 1 and the retainer 2, at least in a direction which could lead to further tightening, is blocked. Thus, the first and second distal surfaces 2.1, 1.2 may be spaced away from each other when the needle hub 1 is secured onto the retainer 2 and do not frictionally engage each other so as to reduce the area of the frictional surface between the needle hub 1 and the retainer 2. Thus, the risk of the needle hub 1 getting jammed to the retainer 2 is minimized.

The audible and/or tactile feedback may additionally indicate that the proximal tip 1.1.1 of the needle 1.1 has pierced the septum 3.1 on the distal opening of the medical ampoule 3. Thus, the needle 1.1 is in fluid communication with the interior of the medical ampoule 3 containing the dose of the medicament M and the medical device D is ready to be used for an injection.

In an exemplary embodiment, a window 2.3 may be formed in a portion of the retainer 2 proximal to the threaded surface, and the medical ampoule 3 is preferably made from a transparent material. Thus, the amount of the medicament M left in the medical ampoule 3 may be seen through the window 2.3.

Figure 3 shows an isometric view of another exemplary embodiment of the needle assembly A according to the present invention. In this exemplary embodiment, a plurality of projections 5 is formed on the shoulder 2.2 of the retainer 2, and a plurality of notches 6 are formed on a proximal end of the needle hub 1. Those of skill in the art will understand that design variations may be utilized, e.g., a needle hub 1 with a single notch 6 or the plurality of notches may be utilized with a retainer with a single projection 5 or the plurality of projections 5.

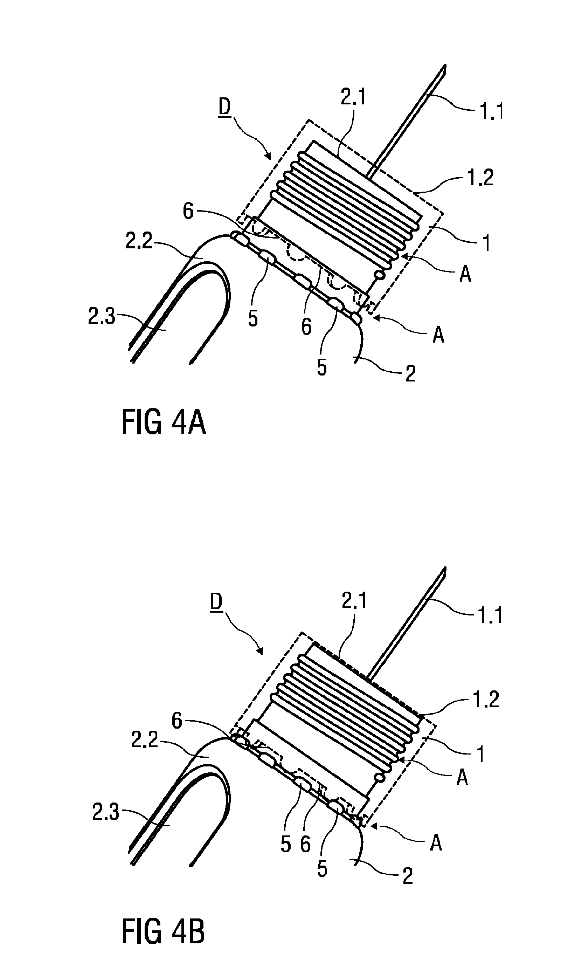

Figure 4A and 4B show isometric views of an exemplary embodiment of the needle assembly A according to the present invention. A first set of projections 5 is formed on the shoulder 2.2 of the retainer 2. A second set of projections is formed on a proximal end of the needle hub 1 and consecutive projections are separated by notches 6. When the needle hub 1 is rotated relative to the retainer 2, a first feedback (e.g., tactile and/or audible) is provided when one of the projections 5 of the first plurality of projections engages a notch 6 on the needle hub 1 and abuts one of the second plurality of projections on the needle hub 1. When the first feedback is provided, the user receives an indication that the needle hub 1 is properly secured to the retainer 2 and that further tightening is unnecessary.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

### List of References

- 1: needle hub
- 1.1: needle
- 1.1.1: proximal tip
- 1.2: second distal surface
- 2: retainer
- 2.1: first distal surface
- 2.2: shoulder
- 2.3: window
- 3: medical ampoule
- 3.1: septum
- 4: boot
- 5: projection
- 6: notch
- A: needle assembly
- D: medical device
- M: medicament
- X: axis

## Claims

1. A needle assembly (A) comprising:
a needle hub (1) having a coupling mechanism adapted to engage corresponding coupling mechanism of a retainer (2); and
a notch (6) formed in a proximal end of the needle hub (1), wherein the notch (6) is adapted to engage a projection (5) on the retainer (2).

2. The needle assembly (A) according to claim 1, wherein a shape of the projection (5) corresponds to a shape of the notch (6).

3. The needle assembly (A) according to claims 1 or 2, wherein a feedback is provided when the projection (5) engages the notch (6).

4. The needle assembly (A) according to claim 3, wherein the feedback is at least one of a tactile feedback and an audible feedback.

5. The needle assembly (A) according to any one of the preceding claims, wherein a plurality of notches (6) are formed in the proximal end of the needle hub (1).

6. The needle assembly (A) according to any one of the preceding claims, wherein a plurality of projections (5) are formed on the retainer (2).

7. The needle assembly (A) according to claim 6, wherein the plurality of projections (5) are disposed at regular intervals.

8. The needle assembly (A) according to any one of the preceding claims wherein the notch (6) is an inverted U-shape.

9. The needle assembly (A) according to any one of the preceding claims wherein the notch (6) is triangular.

10. The needle assembly (A) according to any one of the preceding claims wherein the notch (6) includes a surface adapted to prevent movement of the projection in a first direction.

11. The needle assembly (A) according to any one of the preceding claims, wherein the retainer (2) is an injection device.
